# EUROPEAN PATENT SPECIFICATION

(11) **EP 2 194 981 B1**
(45) Date of publication and mention of the grant of the patent: **23.11.2011**
(21) Application number: 08835050.9
(22) Date of filing: 02.10.2008
(51) Int. Cl.: A61K 31/4184, A61P 33/06, A61K 45/06

(54) **BISBENZIMIDAZOLES AS ANTIMALARIAL AGENTS**
BISBENZIMIDAZOLE UND MITTEL GEGEN MALARIA
BISBENZIMIDAZOLES EN TANT QU'AGENTS ANTIPALUDIQUES

(30) Priority: 05.10.2007 EP 07117987
(43) Date of publication of application: 16.06.2010
(73) Proprietor: Université de Mons-Hainaut, 7000 Mons (BE); Xavier University of Louisiana, New Orleans LA 70125 (US)
(72) Inventor: VANDEN EYNDE, Jean Jacques, B-7000 Mons (BE); PIETKA, Aurélie, B-7321 Blaton (BE); HUANG, Tien, Metairie LA 70005 (US); MAYENCE, Annie, B-7000 Mons (BE)
(74) Representative: Molnia, David
(86) International application number: PCT/EP2008/063224
(87) International publication number: WO 2009/043902

(56) References cited:
- WO-A-95/08540
- DE-A1- 19 853 944
- BELL C A ET AL: "STRUCTURE-ACTIVITY RELATIONSHIPS OF ANALOGS OF PENTAMIDINE AGAINST PLASMODIUM FALCIPARUM AND LEISHMANIA MEXICANA AMAZONENSIS" ANTIMICROBIAL AGENTS AND CHEMOTHERAPY, AMERICAN SOCIETY FOR MICROBIOLOGY, WASHINGTON, DC, US, vol. 34, no. 7, 1 July 1990 (1990-07-01), pages 1381-1386, XP008013577 ISSN: 0066-4804
- DONKOR I O ET AL: "Trypanocidal activity of dicationic compounds related to pentamidine" EUROPEAN JOURNAL OF MEDICINAL CHEMISTRY, EDITIONS SCIENTIFIQUE ELSEVIER, PARIS, FR, vol. 36, no. 6, 1 June 2001 (2001-06-01), pages 531-538, XP004372864 ISSN: 0223-5234

## Description

### Field of the invention

The present invention relates to antimalarial agents. In particular, the present invention relates to bisbenzimidazoles for use in the treatment of malaria.

### Background of the invention

Malaria is a parasitic disease which is caused by various species of Plasmodium protozoa. Together with AIDS and TB, malaria is responsible for largest number of deaths annually. The high rate of mortality associated with malaria can be attributed to the increasing cases of resistance of *Plasmodium falciparum,* the most deadly of the four human infecting malarial parasites, to the contemporary antimalarial drugs. All attempts to develop vaccines against P. falciparum have failed so far. Therefore, therapies and preventive measures against malaria are confined to drugs. Various classes of antimalarial drugs exist. The most widely used are the quinoline antimalarials, e.g. chloroquine which has been an especially effective drug for both prophylaxis and therapy. However, resistance to many of the currently available antimalarial drugs is spreading rapidly, threatening people in areas where malaria is endemic. Reports of multi-drug resistant strains of malaria parasites render the search for new antimalarial agents especially urgent.

The limitations of the current antimalarial chemotherapeutic arsenal underscore the need for new drugs in this therapeutic area.

It is the aim of the present invention to provide compounds effective in the treatment of malaria to overcome some of the above-mentioned drawbacks.

### Summary of the invention

The present invention relates to bisbenzimidazoles which are useful in the treatment and/or prevention of malaria, in particular Plasmodium falciparum malaria.

Accordingly, the present invention concerns a compound of formula I for use in the treatment of malaria. In particular, the invention concerns the use of at least one compound of Formula I or a pharmaceutically acceptable salt or solvate thereof for the preparation of a medicament for the prevention and/or the treatment of malaria, 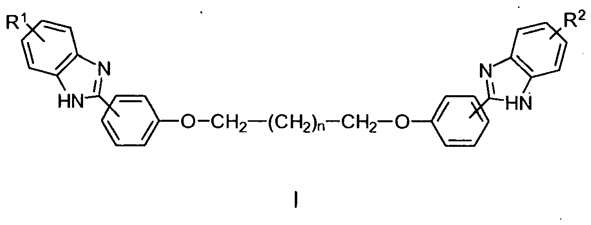 wherein n is an integer selected from 0 to 10, and
R¹ and R² are each independently selected from hydrogen, halogen, hydroxyl, alkyl, alkoxy, amino, alkylamino, alkylcarbonylamino, alkylcarbonyloxy, formyl, alkylcarbonyl, alkyloxycarbonyl, halocarbonyl, haloalkyl, haloalkoxy, carbamoyl, cyano, nitro, sulfo, or a carboxyl group.

The present invention also relates to compounds for use in a method for the therapeutic and/or prophylactic treatment of malaria in a subject in need of such treatment comprising administering to the subject a therapeutically effective amount of a compound of formula I or a pharmaceutically acceptable salt or solvate thereof.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### Detailed description

When describing the compounds for use in the invention, the terms used are to be construed in accordance with the following definitions, unless a context dictates otherwise.

The term "halo" or "halogen" as a group or part of a group is generic for fluoro, chloro, bromo, iodo.

The term "nitro" as used herein refers to the group -NO₂.

The term "cyano" as used herein refers to the group -CN.

The term "alkyl" by itself or as part of another substituent refers to a hydrocarbyl radical of Formula CₙH₂ₙ₊₁ wherein n is a number greater than or equal to 1. Generally, alkyl groups of this invention comprise from 1 to 24 carbon atoms, for example 1 to 20 carbon atoms, for example 1 to 18 carbon atoms, for example 1 to 12 carbon atoms, for example 1 to 8 carbon atoms, for example 1 to 6 carbon atoms, preferably from 1 to 4 carbon atoms, more preferably from 1 to 3 carbon atoms, still more preferably 1 to 2 carbon atoms. Alkyl groups may be linear or branched and may be substituted as indicated herein. When a subscript is used herein following a carbon atom, the subscript refers to the number of carbon atoms that the named group may contain. Thus, for example, C₁₋₄alkyl means an alkyl of one to four carbon atoms. C₁₋₆alkyl includes all linear, or branched alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers.

The term "haloalkyl" alone or in combination, refers to an alkyl radical having the meaning as defined above wherein one or more hydrogens are replaced with a halogen as defined above. Examples of such haloalkyl radicals include chloromethyl, 1-bromoethyl, fluoromethyl, difluoromethyl, trifluoromethyl, 2,2,2-trifluoroethyl

The term "hydroxyl" or "hydroxy" as used herein refers to the group -OH.

The term "alkoxy" or "alkyloxy" as used herein refers to a radical having the Formula -OR^{a} wherein R^{a} is alkyl. Preferably, alkoxy is C₁₋₆ alkoxy, more preferably alkoxy is C₁₋₄ alkoxy. Examples of suitable alkoxy include methoxy, ethoxy, propoxy, isopropoxy, butoxy, isobutoxy, sec-butoxy, tert-butoxy, pentyloxy and hexyloxy. Where the oxygen atom in an alkoxy group is substituted with sulfur, the resultant radical is referred to as thioalkoxy.

The term "haloalkoxy" alone or in combination refers to a group of Formula -O-R^{b} wherein R^{b} is haloalkyl as defined herein. Examples of suitable haloalkoxy include fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy, 2,2,2-trichloroethoxy, trichloromethoxy, 2-bromoethoxy, pentafluoroethyl, 3,3,3-trichloropropoxy, 4,4,4-trichlorobutoxy.

The term "alkylcarbonyl" refers to a group of formula -COR^{c}, wherein R^{c} is C₁₋₆alkyl. Said alkylcarbonyl can be exemplified by acetyl, propionyl, butyryl, valeryl and pivaloyl.

The term "amino" refers to the group -NH₂.

The term "alkylamino" by itself or as part of another substituent refers to a group consisting of an amino group attached to one or two independently selected and optionally substituted alkyl groups, i.e., alkyl amino refers to -N(R^{h})(Rⁱ) wherein R^{h} and Rⁱ are each independently selected from hydrogen or alkyl. Alkylamino includes mono-lower alkyl amino group (e.g. mono-C₁₋₆alkylamino group such as methylamino and ethylamino), di-lower alkylamino group (e.g. di-C₁₋₆alkylamino group such as dimethylamino and diethylamino). Examples of suitable alkylamino groups also include n-propylamino, isopropylamino, n-butylamino, isobutylamino, sec-butylamino, tertbutylamino, pentylamino, n-hexylamino, di-n-propylamino, diisopropylamino, ethylmethylamino, methyl-n-propylamino, methyl-i-propylamino, n-butylmethylamino, i-butylmethylamino, t-butylmethylamino, ethyl-n-propylamino, ethyl-i-propylamino, n-butylethylamino, i-butylethylamino, t-butylethylamino, di-n-butylamino, di-i-butylamino, methylpentylamino, methylhexylamino, ethylpentylamino, ethylhexylamino, propylpentylamino, propylhexylamino.

The term "carboxy" or "carboxyl" refers to the group -CO₂H. Thus, a carboxyalkyl is an alkyl group as defined above having at least one substituent that is -CO₂H.

The term "alkoxycarbonyl" or "alkyloxycarbonyl" refers to a carboxy group linked to an alkyl radical i.e. to form -C(=O)OR^{d}, wherein R^{d} is alkyl as defined above.

The term "alkylcarbonyloxy" refers to a group of Formula -O-C(=O)R^{d} wherein R^{d} is alkyl as defined above.

The term "alkylcarbonylamino" refers to a group of Formula -NH(C=O)R^{m} or - NR^{o}(C=O)R^{m}, wherein R^{m} and R^{o} are each independently alkyl or substituted alkyl.

The term "carbamoyl" (aminocarbonyl) refers to the group -(C=O)-NH₂.

The term "alkylcarbamoyl" refers to a group of Formula -(C=O)-NR^{k}R^{m} wherein R^{k} is hydrogen or alkyl or substituted alkyl as defined herein, and R^{m} is alkyl or substituted alkyl as defined herein. The term encompasses "N-alkylcarbamoyl" wherein R^{k} is hydrogen and R^{m} is alkyl or substituted alkyl, and "N,N-dialkylcarbamoyl" wherein R^{k} and R^{m} are each independently alkyl or substituted alkyl.

The term "sulfo" or "sulfonic acid" refers to a group -SO₃H.

The term "optionally substituted alkyl" refers to an alkyl group optionally substituted with one or more substituent(s) (for example 1, 2, 3 or 4 substituent(s) at any available point of attachment). Unless stated otherwise, examples of suitable substituents include halogen, hydroxyl, oxo, nitro, amido, carboxy, amino, cyano, haloalkoxy, and haloalkyl.

The present invention includes compounds for use in a method of treating or preventing malaria comprising administering to a subject in need thereof an antimalarial effective amount of a compound of Formula I or a pharmaceutically acceptable salt, or solvate thereof.

In a particular embodiment , the invention concerns the use of at least one compound of Formula I or a pharmaceutically acceptable salt or solvate thereof for the preparation of a medicament for the prevention and/or the treatment of malaria, wherein n is an integer selected from 0, 1, 2, 3, 4, 5, 6, 7, 8, 9 or 10, and R¹ and R² are each independently selected from hydrogen, halogen, hydroxyl, C₁₋₆alkyl, C₁₋₂₄alkoxy, amino, C₁₋₂₄alkylamino, C₁₋₂₄alkylcarbonylamino, C₁₋₂₄alkylcarbonyloxy, formyl, C₁₋₂₄alkylcarbonyl, C₁₋₂₄alkyloxycarbonyl, halocarbonyl, halo C₁₋₆alkyl, carbamoyl, C₁₋₂₄alkylcarbamoyl, cyano, nitro, sulfo, or a carboxyl group. In an embodiment, n is 0, 1, 2, 3, 4, 5, 6 or 7.

Preferably, the invention concerns at least one compound of Formula I or a pharmaceutically acceptable salt or solvate thereof for use in the prevention and/or the treatment of malaria, wherein n is an integer selected from 0, 1, 2, 3, 4, 5, 6, 7, 8, 9 or 10, and R¹ and R² are each independently selected from hydrogen, halogen, hydroxyl, C₁₋₄alkyl, C₁₋₁₈alkoxy, amino, C₁₋₁₈alkylamino, C₁₋₁₈alkylcarbonylamino, C₁₋₁₈alkylcarbonyloxy, formyl, C₁₋₁₈alkylcarbonyl, C₁₋₁₈alkyloxycarbonyl, halocarbonyl, haloC₁₋₄alkyl, carbamoyl, C₁₋₁₈alkylcarbamoyl, cyano, nitro, sulfo, or a carboxyl group, preferably, R¹ and R² are each independently selected from hydrogen, halogen, hydroxyl, C₁₋₄alkyl, C₁₋₁₂alkoxy, amino, C₁₋₁₂alkylamino, C₁₋₁₂alkylcarbonylamino, C₁₋₁₂alkylcarbonyloxy, formyl, C₁₋₁₂alkylcarbonyl, C₁₋₁₂alkyloxycarbonyl, halocarbonyl, haloC₁₋₄alkyl, carbamoyl, C₁₋₁₂alkylcarbamoyl, cyano, nitro, sulfo, or a carboxyl group and n is 0, 1, 2, 3, 4, 5, 6 or 7.

In another embodiment, R¹ and R² are each independently selected from hydrogen, hydroxyl, halogen, alkyl, alkoxy, haloalkyl, haloalkoxy, alkyloxycarbonyl, alkylcarbonyl, amino, alkylamino, cyano, nitro or a carboxyl group. The groups of formula 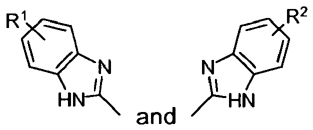 can be independently linked in para, ortho or meta position on the phenyl moiety with respect to the ether bond. In an embodiment, both groups are in para. In another embodiment, both groups are in ortho. In yet another embodiment, both groups are in meta. Preferably both groups are in para, and n is selected from 1, 2, 3, 4, 5, 6 or 7.

Examples of suitable compounds for use in the present invention can be selected from the group comprising 2,2' - [1,3-propanediylbis(oxy-1,4-phenylene)]bis-1 H-benzimidazole, 2,2' - [1,4-butanediylbis(oxy-1,4-phenylene)]bis-1H-benzimidazole, 2,2' - [1,5-pentanediylbis(oxy-1,4-phenylene)]bis-1 H-benzimidazole, 2,2' - [1,6-hexanediylbis(oxy-1,4-phenylene)]bis-1H-benzimidazole, 2,2' - [1,7-heptanediylbis(oxy-1,4-phenylene)]bis-1H-benzimidazole, 2,2' - [1,5-pentanediylbis(oxy-1,3-phenylene)]bis-1H-benzimidazole, 2,2' - [1,5-pentanediytbis(oxy-1,2-phenylene)]bis-1H-benzimidazole, 2,2'-[1,5-pentanediylbis(oxy-1,4-phenylene)]-1H-benzimidazole-5-carboxylic acid; 2,2'-[1,2-ethanediylbis(oxy-1,4-phenylene)]bis-5-nitro-1H-benzimidazole; 2,2'-[1,2-ethanediylbis(oxy-1,4-phenylene)]bis-5-chloro-1H-benzimidazole; 2,2'-[1,3-propanediylbis(oxy-1,4-phenylene)]bis-5-hydroxy-1H-benzimidazole; 2,2'-[1,7-heptanediylbis(oxy-1,4-phenylene)]bis-5-methoxy-1H-benzimidazole; 2,2'-[1,7-heptanediylbis(oxy-1,3-phenylene)]bis-5-methyl-1H-benzimidazole; 2,2'-[1,5-pentanediylbis(oxy-1,3-phenylene)]bis-5-amino-1H-benzimidazole; 2,2'-[1,5-pentanediylbis(oxy-1,3-phenylene)]bis-5-cyano-1 H-benzimidazole; 2,2' - [1,5-pentanediylbis(oxy-1,4-phenylene)]bis-5-trifluoromethoxy-1H-benzimidazole; 2,2' - [1,3-propanediylbis(oxy-1,2-phenylene)]bis-5-tertiobutyl-1H-benzimidazole; 2,2' - [1,3-pentanediylbis(oxy-1,3-phenylene)]bis-5-tertiobutyl-1H-benzimidazole, 2,2'-[1,3-propanediylbis(oxy-1,2-phenylene)]bis-5-bromo-1H-benzimidazole, 2,2'-[1,4-butanediylbis(oxy-1,3-phenylene)]bis-5-bromo-1H-benzimidazole, 2,2'-[1,4-butanediylbis(oxy-1,3-phenylene)]bis-5-diethylamino-1H-benzimidazole, 2,2'-[1,2-ethanediylbis(oxy-1,4-pheny)ene)]bis-5-propoxy-1H-benzimidazole, diethyl 2,2'-[1,2-ethanediylbis(oxy-1,4-pheny)ene)]bis-1H-benzimidazole-5-carboxylate, 2,2'-[1,7-heptanediylbis(oxy-1,3-phenylene)]bis-5-dodecoxy-1H-benzimidazole, dioctyl 2,2'-[1,5-pentanediylbis(oxy-1,2-phenylene)]bis-1 H-benzimidazole-5-carboxylate.

In an embodiment, the compound of formula I is to be administered in combination with at least one additional drug selected from the group consisting of chloroquine, artemesin, qinghaosu, 8-aminoquinoline, amodiaquine, arteether, artemether, artemisinin, artesunate, artesunic acid, artelinic acid, atovoquone, azithromycine, biguanide, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, primaquine, proguanil, pyrimethamine, pyronaridine, quinine, quinidine, quinacrineartemisinin, sulfonamides, sulfones, sulfadoxine, sulfalene, tafenoquine, tetracycline, tetrandine, triazine, salts or mixture thereof.

In an embodiment, the present invention concerns the compounds of formula I, for use in the treatment of drug-resistant malaria.

Preferably, said malaria is selected from *Plasmodium falciparum* malaria, *P*. *vivax* malaria, *P. ovale* malaria, or *P*. *malariae* malaria.

It was found by the invention that the compounds as defined above are particularly useful to prevent or treat an infection of a malarial parasite in a subject and/or for preventing, treating and/or alleviating complications and/or symptoms associated therewith and can then be used in the preparation of a medicament for the treatment and/or prevention of such disease.

Also described herein is administering to a subject in need of such treatment a therapeutically effective amount of a compound of formula I or a pharmaceutically acceptable salt or solvate thereof.

The term "malaria" as used herein refers to an infectious disease, also known as ague or marsh fever, typically caused by a protistan parasite of the genus Plasmodium, suitably, *P. falciparum, P. vivax, P. ovale* or *P. malariae.* This parasite is transmitted primarily by female Anopheles mosquitoes. Plasmodium invades and consumes the red blood cells of its hosts, which leads to symptoms including fever, anemia, and in severe cases, a coma potentially leading to death.

In an embodiment, the invention comprises compounds for use in treating a subject that has been infected with *Plasmodium falciparum.* In an embodiment, the invention comprises compounds for use in treating a subject that has been infected with *P. vivax.* In an embodiment, the invention comprises compounds for use in treating a subject that has been infected with *P. ovale.* In an embodiment, the invention comprises compounds for use in treating a subject that has been infected with *P. malariae.*

In an embodiment, the compound of Formula I is to be administered after the subject has been exposed to the malaria parasite. In another embodiment, the compound of Formula I is to be admnistered before the subject travels to a country where malaria is endemic.

In another embodiment, the malaria parasite is a drug-resistant malarial strain.

The term a "therapeutically effective amount", "effective amount" or a "sufficient amount " of a compound of Formula I is a quantity sufficient to, when administered to the subject, including a mammal, for example a human, effect beneficial or desired results, including clinical results, and, as such, an "effective amount" or synonym thereto depends upon the context in which it is being applied.

In the context of disease, therapeutically effective amounts of the compounds of Formula I are for use to treat, modulate, attenuate, reverse, or effect malaria in a mammal. An "effective amount" is intended to mean that amount of a compound that is sufficient to treat, prevent or inhibit malaria or a disease associated with malaria. In some suitable embodiments, malaria or the disease or disorder associated with malaria is caused by a Plasmodium parasite, suitably, *P. falciparum, P. vivax, P. ovale* or *P. malariae,* thus it is the amount sufficient to, when administered to the subject, including a mammal, e.g., a human, to treat, prevent or inhibit malaria or a disease or a disorder associated with malaria or a malarial parasite, e.g. Plasmodium parasite. The amount of a given compound that will correspond to such an amount will vary depending upon various factors, such as the given drug or compound, the pharmaceutical formulation, the route of administration, the type of disease or disorder, the identity of the subject or host being treated, and the like, but can nevertheless be routinely determined by one skilled in the art. Also, as used herein, a "therapeutically effective amount" of a compound of Formula I is an amount which prevents, inhibits, suppresses or reduces malaria (e.g., as determined by clinical symptoms or the amount of malarial parasites, e.g., Plasmodium organisms) in a subject as compared to a control. As defined herein, a therapeutically effective amount of a compound of Formula I may be readily determined by one of ordinary skill by routine methods known in the art. The skilled artisan will appreciate that certain factors may influence the dosage required to effectively treat a subject, or prevent a subject, from being afflicted with malaria and these factors include the severity of the disease or disorder, previous treatments, the general health and/or age of the subject and other diseases present.

Moreover, a "treatment" or "prevention" regime of a subject with a therapeutically effective amount of the compound of Formula I may consist of a single administration, or alternatively comprise a series of applications. For example, the compound of Formula I may be administered at least once a week. However, in another embodiment, the compound of Formula I may be administered to the patient from about one time per week to about once daily for a given treatment. The length of the treatment period depends on a variety of factors, such as the severity of the disease, the age of the patient, the concentration and the activity of the compounds of Formula I, or a combination thereof. It will also be appreciated that the effective dosage of the compound for use in the treatment or prophylaxis may increase or decrease over the course of a particular treatment or prophylaxis regime. Changes in dosage may result and become apparent by standard diagnostic assays known in the art. In some instances, chronic administration may be required. The compounds of Formula I may be administered before, during or after exposure to malaria or malarial parasite, e.g. Plasmodium parasite.

As used herein, and as well understood in the art, "treatment" is an approach for obtaining beneficial or desired results, including clinical results. Beneficial or desired clinical results can include alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

The term "prevention" or "prophylaxis", or synonym thereto, as used herein refers to a reduction in the risk or probability of a patient becoming afflicted with malaria or manifesting a symptom associated with malaria.

The term "subject" as used herein refers to a mammal. The individual will preferably be a human, but may also be a domestic livestock, laboratory or pet animal.

As used in the specification and the appended claims, the singular forms "a", "an," and "the" include plural referents unless the context clearly dictates otherwise. By way of example, "a compound" means one compound or more than one compound.

Whenever used in the present invention the term "compounds for use in the invention" or a similar term is meant to include the compounds of general Formula I, N-oxides, salts, solvates, hydrates, tautomeric forms, esters, as well as their quaternized nitrogen analogues. The N-oxide forms of said compounds are meant to comprise compounds wherein one or several nitrogen atoms are oxidized to the so-called N-oxide.

The compounds of the invention may be in the form of pharmaceutically acceptable salts, as generally described below. Some preferred, examples of suitable pharmaceutically acceptable organic and/or inorganic acids are as hydrochloric acid, hydrobromic acid, sulfuric acid, nitric acid, acetic acid and citric acid, as well as other pharmaceutically acceptable acids known per se (for which reference is made to the prior art referred to below).

When the compounds of the invention contain an acidic group as well as a basic group the compounds of the invention may also form internal salts, and such compounds are within the scope of the invention. When the compounds of the invention contain a hydrogen-donating heteroatom (e.g. NH), the invention also covers salts and/or isomers formed by transfer of said hydrogen atom to a basic group or atom within the molecule.

Pharmaceutically acceptable salts of the compounds of formula I include the acid addition and base salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts. For a review on suitable salts, see Handbook of Pharmaceutical Salts: Properties, Selection, and Use by Stahl and Wermuth (Wiley-VCH, 2002) Pharmaceutical composition for use in the present invention preferably comprises an effective amount of a compound of formula I and a suitable pharmaceutical acceptable carrier.

The preparations may be prepared in a manner known per se, which usually involves mixing the at least one compound according to the invention with the one or more pharmaceutically acceptable carriers, and, if desired, in combination with other pharmaceutical active compounds, when necessary under aseptic conditions. Reference is made to US-A-6,372,778, US-A-6,369,086, US-A-6,369,087 and US-A-6,372,733 and the further prior art mentioned above, as well as to the standard handbooks, such as the latest edition of Remington's Pharmaceutical Sciences.

Generally, for pharmaceutical use, the compounds may be formulated as a pharmaceutical preparation comprising at least one compound of formula I and at least one pharmaceutically acceptable carrier, diluent or excipient and/or adjuvant, and optionally one or more further pharmaceutically active compounds.

The pharmaceutical preparations of the invention are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use. Generally, such unit dosages will contain between 1 and 1000 mg, and usually between 5 and 500 mg, of the at least one compound of the invention, e.g. about 10, 25, 50, 100, 200, 300 or 400 mg per unit dosage.

The compounds can be administered by a variety of routes including the oral, ocular, rectal, transdermal, subcutaneous, intravenous, intramuscular or intranasal routes, depending mainly on the specific preparation used. The compound of formula I will generally be administered in an "effective amount", by which is meant any amount of a compound of the Formula I that, upon suitable administration, is sufficient to achieve the desired therapeutic or prophylactic effect in the subject to which it is administered. Usually, depending on the condition to be prevented or treated and the route of administration, such an effective amount will usually be between 0.01 to 1000 mg per kilogram body weight of the patient per day, more often between 0.1 and 500 mg, such as between 1 and 250 mg, for example about 5, 10, 20, 50, 100, 150, 200 or 250 mg, per kilogram body weight of the patient per day, which may be administered as a single daily dose, divided over one or more daily doses. The amount(s) to be administered, the route of administration and the further treatment regimen may be determined by the treating clinician, depending on factors such as the age, gender and general condition of the patient and the nature and severity of the disease/symptoms to be treated. Reference is again made to US-A-6,372,778, US-A-6,369,086, US-A-6,369,087 and US-A-6,372,733 and the further prior art mentioned above, as well as to the standard handbooks, such as the latest edition of Remington's Pharmaceutical Sciences.

For an oral administration form, the compound of formula I can be mixed with suitable additives, such as excipients, stabilizers or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, the compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the compounds of the invention or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant.

For subcutaneous or intravenous administration, the compounds of formula I, if desired with the substances customary therefore such as solubilizers, emulsifiers or further auxiliaries are brought into solution, suspension, or emulsion. The compounds of formula I can also be lyophilized and the lyophilizates obtained used, for example, for the production of injection or infusion preparations. Suitable solvents are, for example, water, physiological saline solution or alcohols, e.g. ethanol, propanol, glycerol, in addition also sugar solutions such as glucose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, the formulations may be prepared by mixing the compounds of formula I with a suitable non-irritating excipient, such as cocoa butter, synthetic glyceride esters or polyethylene glycols, which are solid at ordinary temperatures, but liquefy and/or dissolve in the rectal cavity to release the drug.

The compounds may be formulated for local effect, for instance topical or non-adsorbent applications.

The compounds of formula I or the above-mentioned pharmaceutical compositions may also be used in combination with one or more other therapeutically useful substances selected from the group comprising antimalarials like quinolines (quinine, chloroquine, amodiaquine, mefloquine, primaquine, tafenoquine); peroxide antimalarials (artemisinin, artemether, artesunate); pyrimethamine-sulfadoxine antimalarials (e.g. Fansidar); hydroxynaphtoquinones (e.g. atovaquone); acroline-type antimalarials (e.g. pyronaridine); and antiprotozoal agents such as ethylstibamine, hydroxystilbamidine, pentamidine, stilbamidine, quinapyramine, puromycine, propamidine, nifurtimox, melarsoprol, nimorazole, nifuroxime, aminitrozole

In particular, the present invention also concerns compounds for use in a method of treating or preventing malaria comprising administering to a subject in need thereof an anti-malarial effective amount compound selected from a compound of Formula I, pharmaceutically acceptable salts, solvates thereof, in combination with at least one additional drug selected from the group consisting of chloroquine, artemesin, qinghaosu, 8-aminoquinoline, amodiaquine, arteether, artemether, artemisinin, artesunate, artesunic acid, artelinic acid, atovoquone, azithromycine, biguanide, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, primaquine, proguanil, pyrimethamine, pyronaridine, quinine, quinidine, quinacrineartemisinin, sulfonamides, sulfones, sulfadoxine, sulfalene, tafenoquine, tetracycline, tetrandine, triazine or salts thereof.

Also described is a method comprising step of exposing malaria cells to an effective concentration of a compound of the formula I, in combination with at least one additional drug selected from the group described above.

The present invention also concerns a composition for use in preventing or treating malaria comprising: a compound of the formula I in combination with at least one additional drug selected from the group consisting of chloroquine, artemesin, qinghaosu, 8-aminoquinoline, amodiaquine, arteether, artemether, artemisinin, artesunate, artesunic acid, artelinic acid, atovoquone, azithromycine, biguanide, chloroquine, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, primaquine, proguanil, pyrimethamine, pyronaridine, quinine, quinidine, quinacrineartemisinin, sulfonamides, sulfones, sulfadoxine, sulfalene, tafenoquine, tetracycline, tetrandine, triazine or derivatives or mixture thereof.

It was found by the invention that the compounds as defined above are particularly useful to prevent or treat an infection of a malarial parasite in a subject and/or for preventing, treating and/or alleviating complications and/or symptoms associated therewith and can then be used in the preparation of a medicament for the treatment and/or prevention of such disease.

The invention will now be illustrated by means of the following synthetic and biological examples

### Examples

The following general procedures and intermediates were used to prepare the compounds described herein. Compounds for use in the present invention can be prepared as described in Scheme 1. 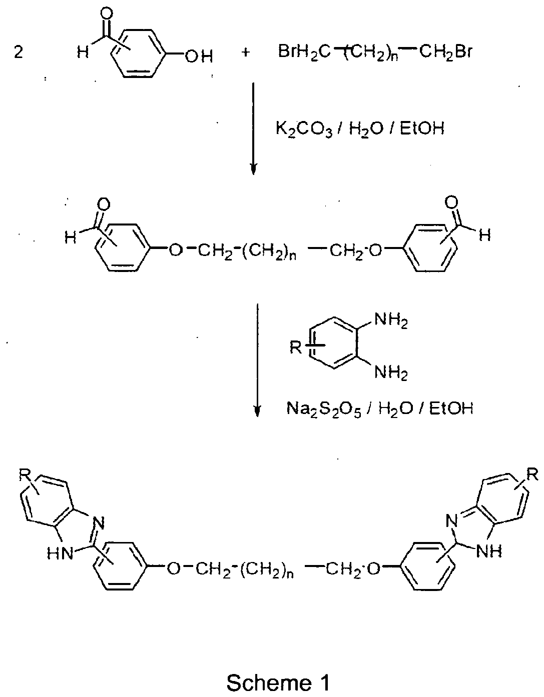 with n= 0 to 10, and each R is independently selected from hydrogen, halogen, hydroxyl, alkyl, alkoxy, amino, alkylamino, alkylcarbonylamino, alkylcarbonyloxy, formyl, alkylcarbonyl, alkyloxycarbonyl, halocarbonyl, haloalkyl, haloalkoxy, carbamoyl, cyano, nitro, sulfo, or a carboxyl group.

For example, each R is independently selected from H, -CO₂H, Cl, F, Br, -OH, -Me, -Et, - nPr, -iPr, tBu, -OMe, -OEt, -OnPr, -OiPr, -OnBu, -OiBu, -OtBu, -O-C₅-H₁₁, -O-C₆H₁₃, -O-C₇H₁₅, -O-C₈H₁₇, -O-C₉H₁₉, -O-C₁₀H₂₁, -O-C₁₁H₂₃, O-C₁₂H₂₅, -O-C₁₃H₂₇, -O-C₁₄H₂₉, ..., - OC₁₈H₃₇, ... -OC₂₄H₄₉, -NH₂, -NH(Me), -N(Me)₂, -N(Et)₂, -NH-CO-Me, -NH-CO-Et, - OCOMe, -OCOEt, -OCOnPr, -OCOiPr, -OCOnBu, -OCOiBu, -OCOtBu, -OCO-C₅H₁₁, - OCO-C₆H₁₃, -OCO-C₇H₁₅, -OCO-C₈H₁₇, -OCO-C₉H₁₉, -OCO-C₁₀H₂₁, -OCO-C₁₁H₂₃, OCO-C₁₂H₂₅, -OCO-C₁₃H₂₇, -OCO-C₁₄H₂₉, ..., -OCOC₁₈H₃₇, ... -OCOC₂₄H₄₉, -CHO, -COMe, - COEt, -CO₂Me, -CO₂Et, -COCl, -CF₃, -CCl₃, -CHF₂, -OCF₃, -CONH₂, -CN, -NO₂, -SO₃H.

### General procedure

A mixture of an hydroxybenzaldehyde (5.12 g; 42 mmol), a dibromoalkane (20 mmol), and potassium carbonate (2.76 g; 20 mmol) in ethanol (10 ml) was heated under reflux for 8 hours. After cooling, the precipitate was filtered and successively washed with water, ethanol and ether. The bisbenzaldehyde intermediate was pure enough to be engaged in the second step.

A mixture of the bisbenzaldehyde intermediate (3 mmol), sodium pyrosulfite (0.76 g; 4 mmol), an ortho- phenylenediamine (6.5 mmol), and water (3 ml) in ethanol (9 ml) was irradiated in a professional microwave oven for 15 min at 140 °C. After cooling the precipitate was filtered and thoroughly washed with water, ethanol, and ether and dried to obtain the compound for use in the invention.

### Intermediates

### Intermediate 1: 4,4'-[1,3-propanediyl(oxy)]bisbenzaldehyde

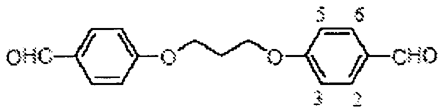

IR (KBr, cm-1): 2952, 1683, 1606, 1578, 1510.
1H NMR (DMSO-d6, ppm): 9.9 (s, 2H, CHO); 7.8 (d, J=7Hz, 4H, C6H4:H2,H6); 7.2 (d, 4H, J=7Hz, C6H4:H3,H5); 4.3 (t, 4H, J=6Hz, O-CH₂-CH₂); 2.3 (m, 2H, J=6Hz, O-CH₂-CH₂).
Mp (°C): 193.2- 196.3 °C. Yield: 78% MW: 284.31.

### Intermediate 2: 4,4'-[1,4-butanediyl(oxy)]bisbenzaldehyde

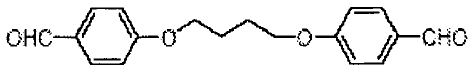

IR (KBr, cm-1): 2943, 1697, 1601, 1580, 1510.
1H NMR (DMSO-d6, ppm): 9.9 (s, 2H, CHO); 7.9 (d, 4H, J=6Hz, C6H4: H2,H6); 7.1 (d, 4H, J=6Hz, C6H4:H3,H5); 4.2 (t, 4H, J=6Hz, O-CH2-CH2); 1.9 (m, 4H, O-CH2-CH2).
Mp (°C): 153- 158°C. Yield: 70% MW: 298.33.

### Intermediate 3: 4,4'-[1,5-pentanediyl(oxy)]bisbenzaldehyde

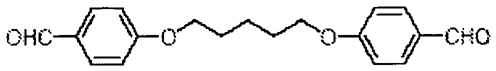

IR (KBr, cm-1): 2945, 1692, 1604, 1576, 1510.
1H NMR (DMSO-d6, ppm): 9.9 (s, 2H, CHO); 7.9 (d, 4H, J=8Hz, C6H4:H2,H6); 7.1 (d, 4H, J= 8Hz, C6H4:H3,H5); 4.2 (t, 4H, J=6Hz, O-CH2-CH2-CH2); 1.8 (m, 4H, J=6Hz, O-CH2-CH2-CH2); 1.5 (m, 2H, J= 6Hz, O-CH2-CH2-CH2).
Mp (°C): 90.7- 94.5°C. Yield: 90% MW : 312.36.

### Intermediate 4: 4,4'-[1,6-hexanediyl(oxy)]bisbenzaldehyde

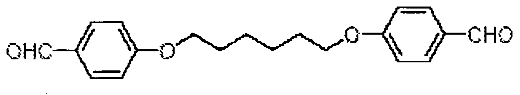

IR (KBr, cm-1): 2946, 2876, 1686, 1599, 1511.
1H NMR (DMSO-d6, ppm): 9.9 (s, 2H, CHO); 7.9 (d, 4H, J=9Hz, C6H4:H2,H6); 7.1 (d, 4H, J=9Hz, C6H4:H3,H5); 4.1 (t, 4H, J=6Hz, O-CH2-CH2-CH2); 1.8 (m, 4H, O-CH2-CH2-CH2); 1.5 (m, 4H, O-CH2-CH2-CH2).
Mp (°C): 111- 116°C. Yield: 84% MW: 326.39.

### Intermediate 5: 4,4'-[1,7-heptane(oxy)]bisbenzaldehyde

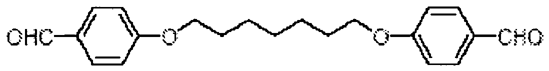

IR (KBr, cm-1): 2945, 1686, 1599, 1573, 1510.
1 H NMR (DMSO-d6, ppm): 9.9 (s, 2H, CHO); 7.9 (d, 4H, J=9Hz, C6H4:H2,H6); 7.1 (d, 4H, J=9Hz, C6H4:H3,H5); 4.1 (t, 4H, J=6Hz, O-CH2-CH2-CH2); 1.8 (m, 4H, O-CH2-CH2-CH2); 1.4 (m, 2H+4H, O-CH2-CH2-CH2).

Mp (°C): 63.8- 65.3°C. Yield: 59% MW: 340.41.

### Intermediate 6: 2,2'-[1,5-pentanediyl(oxy)]bisbenzaldehyde

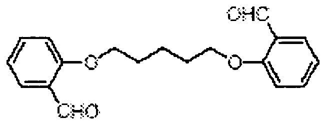

IR (KBr, cm-1): 2947, 2874, 1678, 1601, 1459.
1H NMR (DMSO-d6, ppm): 10.4 (s, 2H, CHO); 7.7 (d, 2H, J=8Hz, C6H4: H6); 7.6 (d, 2H, J=8Hz, C6H4: H4); 7.2 (d, 2H, J=8Hz, C6H4: H3); 7.1 (t, 2H, J=8Hz, C6H4: H5); 4.2 (t, 4H, J=6Hz, O-CH2-CH2-CH2); 1.9 (m, 4H, J=6Hz, O-CH2-CH2-CH2); 1.7 (m, 2H, J=6Hz, O-CH2-CH2-CH2).
Mp (°C): 65.8- 70°C. Yield: 79% MW: 312.36.

### Intermediate 7: 3,3'-[1,5-pentanediyl(oxy)]bisbenzaldehyde

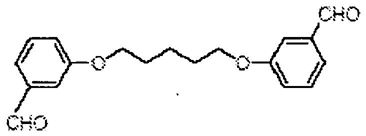

IR (KBr, cm-1): 2940, 2793, 1697, 1603, 1455.
1H NMR (DMSO-d6, ppm): 10 (s, 2H, CHO); 7.6-7.2 (m, 8H, Ph); 4.1 (t, 4H, J=6Hz, O-CH2-CH2-CH2); 1.8 (m, J=6Hz, 4H, O-CH2-CH2-CH2); 1.6 (m, 2H, J=6Hz, O-CH2-CH2-CH2).
Mp (°C): 55.1- 56.9°C. Yield: 37% MW: 312.36.

### Compounds

The compounds were obtained following the general procedure described herein using the corresponding intermediates described herein. The present invention encompasses the use of compounds 1 to 25 and their derivatives, N-oxides, salts, solvates, hydrates, tautomeric forms, analogues, pro-drugs, esters and metabolites, as well as their quaternized nitrogen analogues.

### Compound 1: 2,2' - [1,3-propanediylbis(oxy-1,4-phenylene)]bis-1H-benzimidazole

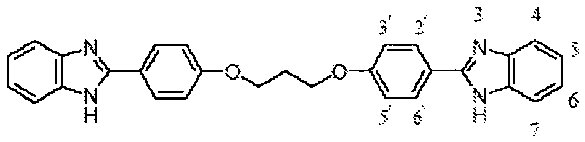

IR (KBr, cm-1): 3631-2667(N-H bz), 1612, 1499, 1451, 1252.
1H NMR (DMSO-d6, ppm): 8.1 (d, 4H, J=9Hz, C6H4: H2',H6'); 7.6 (AA'BB', 4H, benz: H4,H7); 7.2 (AA'BB', 4H, benz: H5,H6); 7.1 (d, 4H, J=9Hz, C6H4: H3',H5'); 4.3 (t, 4H, J=6Hz, O-CH2-CH2); 2.2 (m, 2H, J=6Hz, O-CH2-CH2).
HRMS (ESI-ToF) MH+ C29H25N4O2 : MW experimental: m/z 461.1973. MW calculated: m/z 461.1978.
Mp (°C): > 300°C. Yield: 98%.

### Compound 2: 2,2' - [1,4-butanediylbis(oxy-1,4-phenylene)]bis-1H-benzimidazole

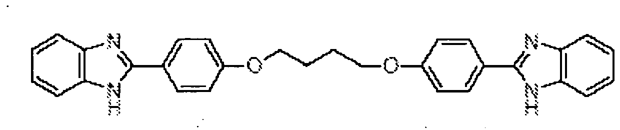

IR (KBr, cm-1): 3631-2364(N-H bz), 1612, 1500, 1439, 1253.
1H NMR (DMSO-d6, ppm): 8.1 (d, 4H, J=9Hz, C6H4: H2',H6'); 7.6 (AA'BB', 4H, benz: H4,H7); 7.2 (AA'BB', 4H, benz: H5,H6); 7.1 (d, 4H, J=9Hz, C6H4: H3',H5 '); 4.2 (t, 4H, J=6Hz, O-CH2-CH2); 2.0 (m, 4H, O-CH2-CH2).
HRMS (ESI-ToF) MH+ C30H27N4O2: MW experimental: m/z 475.2155. MW calculated: m/z 475.2134.
Mp (°C): > 300°C. Yield: 93%.

### Compound 3: 2,2' - [1,5-pentanediylbis(oxy-1,4-phenylene)]bis-1H-benzimidazole

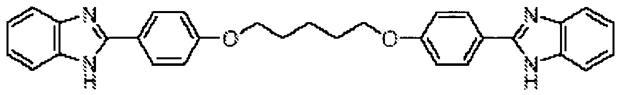

IR (KBr, cm-1): 3623-2400(N-H bz), 1762, 1611, 1500, 1254.
1H NMR (DMSO-d6, ppm): 8.1 (d, 4H, J=9Hz, C6H4: H2',H6'); 7.6 (AA'BB', 4H, benz: H4,H7); 7.2 (AA'BB', 4H, benz: H5,H6); 7.1 (d, 4H, J=9Hz, C6H4: H3',H5'); 4.1 (t, 4H, J=6Hz, O-CH2-CH2-CH2); 1.8 (m, 4H, O-CH2-CH2-CH2); 1.6 (m, 2H, O-CH2-CH2-CH2).
HRMS (ESI-ToF) MH+ C31H29N4O2: MW experimental: m/z 489.2275. MW calculated: m/z 489.2291.
Mp (°C): > 300°C. Yield: 94%.

### Compound 4: 2,2' - [1,6-hexanediylbis(oxy-1,4-phenylene)]bis-1H-benzimidazole

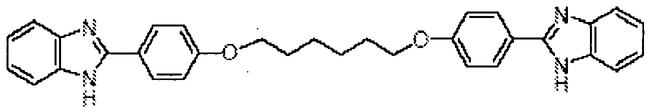

IR (KBr, cm-1): 3570-2756(N-H bz), 1613, 1500, 1451, 1254.
1H NMR (DMSO-d6, ppm): 8.1 (d, 4H, J=9Hz, C6H4: H2',H6'); 7.6 (AA'BB', 4H, benz: H4,H7); 7.2 (AA'BB', 4H, benz: H5,H6); 7.1 (d, 4H, J=9Hz, C6H4: H3',H5'); 4.1 (t, 4H, J=6Hz, O-CH2-CH2-CH2); 1.8 (m, 4H, O-CH2-CH2-CH2); 1.6 (m, 4H, O-CH2-CH2-CH2).
HRMS (ESI-ToF) MH+ C32H31N4O2 MW experimental: m/z 503.2462. MW calculated: m/z 503.2447.
Mp (°C): > 300°C. Yield: 89%.

### Compound 5: 2,2' - [1,7-heptanediylbis(oxy-1,4-phenylene)]bis-1 H-benzimidazole

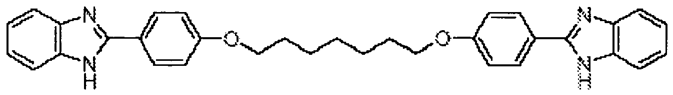

IR (KBr, cm-1): 3632-2757(N-H bz), 1613, 1500, 1451, 1255.
¹H NMR (DMSO-d6, ppm): 8.1 (d, 4H, J=9Hz, C6H4: H2',H6'); 7.6 (AA'BB', 4H, benz: H4,H7), 7.2 (AA'BB', 4H, benz: H5,H6); 7.1 (d, 4H, J=9Hz, C6H4: H3',H5'); 4.0 (t, 4H, J=6Hz, O-CH2-CH2-CH2); 1.8 (m, 4H, O-CH2-CH2-CH2); 1.4 (m, 2H+4H, O-CH2-CH2-CH2).
HRMS (ESI-ToF) MH+ C33H33N4O2: MW experimental: m/z 517.2627; MW calculated: m/z 517.2604.
Mp (°C): 298- 301 °C. Yield: 96%.

### Compound 6: 2,2' - [1,5-pentanediyibis(oxy-1,3-phenylene)]bis-1H-benzimidazole

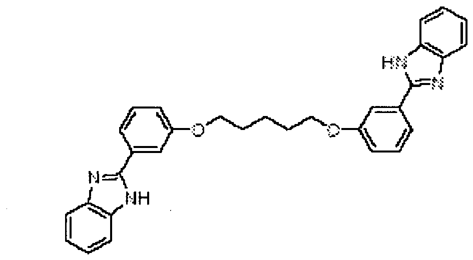

IR (KBr, cm-1): 3705-2642(N-H bz), 1601, 1492, 1450, 1236.
1H NMR (DMSO-d6, ppm): 7.8 (d,s., J=7Hz, 2H+2H, C6H4: H4',H2'); 7.6 (AA'BB', 4H, benz: H4,H7); 7.4 (t, 2H, J=7Hz, C6H4: H5'); 7.1 (d, 2H, J=7Hz, C6H4: H6'); 7.2 (AA'BB',4H, benz: H5,H6); 4.1 (t, 4H, J= 6Hz, O-CH2-CH2-CH2); 1.9 (m, 4H, J= 6Hz, O-CH2-CH2-CH2); 1.5 (m, 2H, J= 6Hz, O-CH2-CH2-CH2).
HRMS (ESI-ToF) MH+ C31H29N4O2 : MW experimental: m/z 489.2271. MW calculated: m/z 489.2291.
Mp (°C): 240- 243°C. Yield: 75%.

### Compound 7: 2,2' - [1,5-pentanediylbis(oxy-1,2-phenylene)]bis-1H-benzimidazole

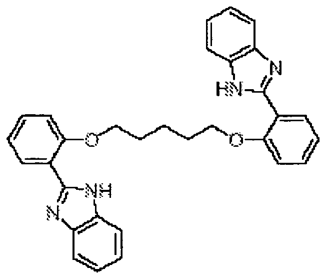

IR (KBr, cm-1): 3652-2285(N-H bz), 1603, 1490, 1455, 1240.
1H NMR (DMSO-d6, ppm): 8.2 (d, J=7Hz, 2H, C6H4: H6'); 7.6 (AA'BB', 4H, benz: H4,H7); 7.4 (t, 2H, J=7Hz, C6H4: H4'); 7.2 (AA'BB', d, 2H+4H, C6H4: H3' benz: H5,H6); 7.1 (t, 2H, J=7Hz, C6H4: H5'); 4.2 (t, 4H, J=6Hz, O-CH2-CH2-CH2); 1.9 (m, 4H, J= 6Hz, O-CH2-CH2-CH2); 1.5 (m, 2H, J= 6Hz, O-CH2-CH2-CH2).
HRMS (ESI-ToF) MH+ C31H29N4O2: MW experimental: m/z 489.2296. MW calculated: m/z 489.2291.
Mp (°C): 190- 193°C. Yield: 74%.

### Compound 8: 2,2'-[1,5-pentanediylbis(oxy-1,4-phenylene)]-1H-benzimidazole-5-carboxylic acid

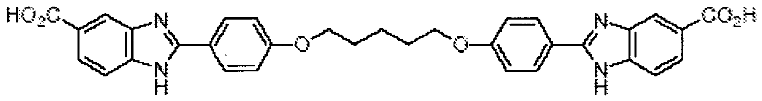

IR (KBr, cm-1): 3691-2362(N-H bz), 1680, 1609, 1505, 1256.
1 H NMR (DMSO-d6, ppm): 13.0 (s, 1 H, COOH); 8.2 (d,s.,4H+2H,J=8Hz, C6H4: H6',H2', benz: H4); 7.8 (d, 2H, J= 8Hz, benz: H6); 7.6 (d, 2H, J= 8Hz, benz: H7); 7.2 (d, 4H, J= 8Hz, C6H4: H5', H3'); 4.1 (t, 4H, J= 6Hz, O-CH2-CH2-CH2); 1.8 (m, 4H, J= 6Hz, O-CH2-CH2-CH2); 1.6 (m, 2H, J=6Hz, O-CH2-CH2-CH2).

HRMS (ESI-ToF) MH+ C33H28N4O6: MW experimental: m/z 577.2091. MW calculated: m/z 577.2087.
Mp (°C): 292- 296°C. Yield: 97%.

### Compounds 9-25 set out in tabulated form in Table 1 are synthesized using similar protocols.

**Table 1**

| **Compound** | **Name** | **Structure** |
|---|---|---|
| **9** | 2,2'-[1,2-ethanediylbis(oxy-1,4-phenylene)]bis-5-nitro-1H-benzimidazole | 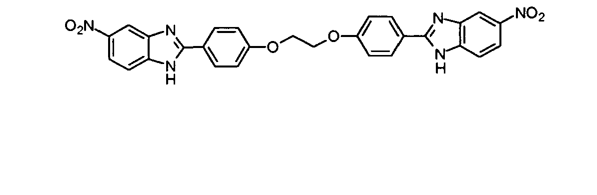 |
| **10** | 2,2'-[1,2-ethanediylbis(oxy-1,4-phenylene)]bis-5-chloro-1 H-benzimidazole | 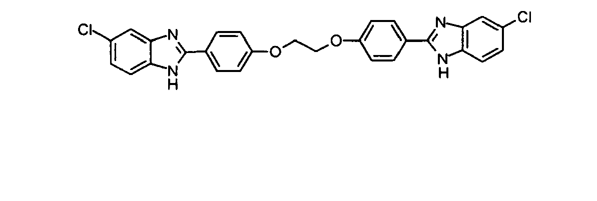 |
| **11** | 2,2'-[1,3-propanediylbis(oxy-1,4-phenylene)]bis-5-hydroxy-1H-benzimidazole | 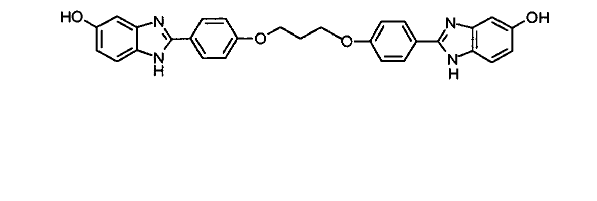 |
| **12** | 2,2'-[1,7-heptanediylbis(oxy-1,4-phenylene)]bis-5-methoxy-1H-benzimidazole | 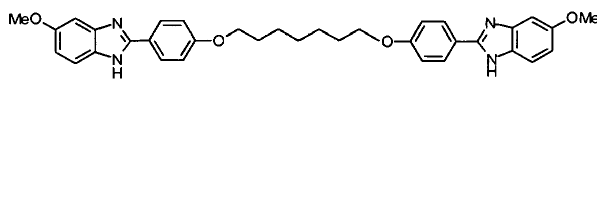 |
| **13** | 2,2'-[1,7-heptanediylbis(oxy-1,3-phenylene)]bis-5-methyl-1 H-benzimidazole | 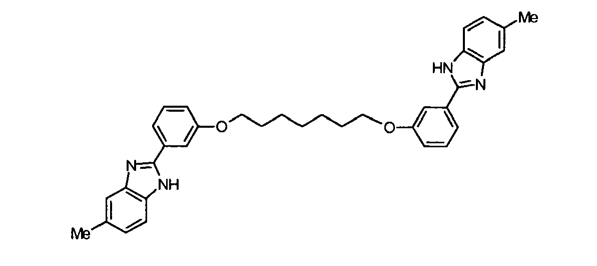 |
| **14** | 2,2'-[1,5-pentanediylbis(oxy-1,3-phenylene)]bis-5-amino-1 H-benzimidazole | 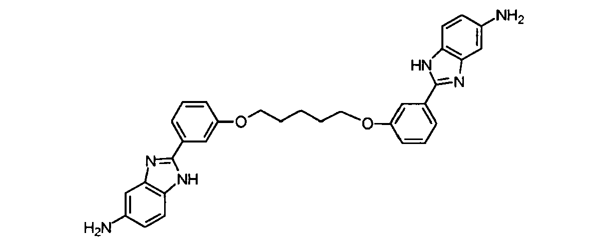 |
| **15** | 2,2'-[1,5-pentanediylbis(oxy-1,3-phenylene)]bis-5-cyano-1H-benzimidazole | 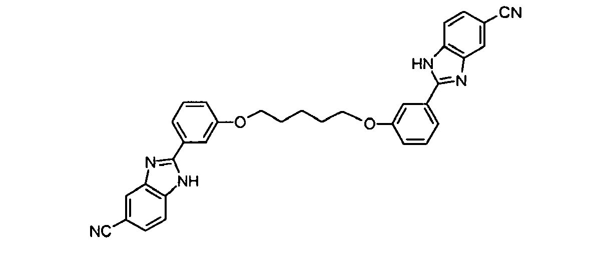 |
| **16** | 2,2' - [1,5-pentanediylbis(oxy-1,4-phenylene)]bis-5-trifluoromethoxy-1 H-benzimidazole | 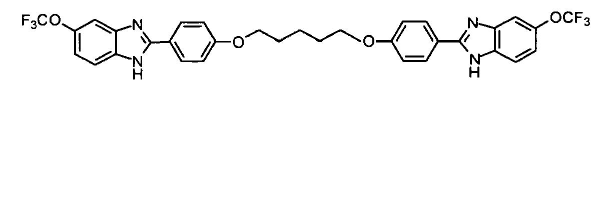 |
| **17** | 2,2' - [1,3-propanediylbis(oxy-1,2-phenylene)]bis-5-tertiobutyl-1 H-benzimidazole | 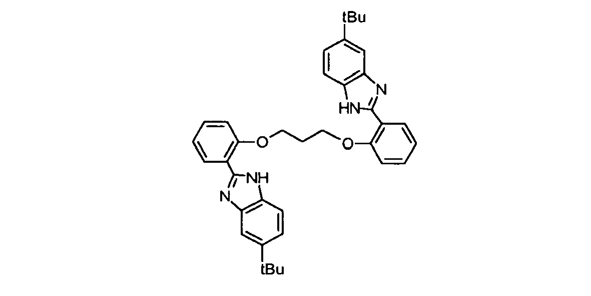 |
| **18** | 2,2' - [1,3-pentanediylbis(oxy-1,3-phenylene)]bis-5-tertiobutyl-1 H-benzimidazole | 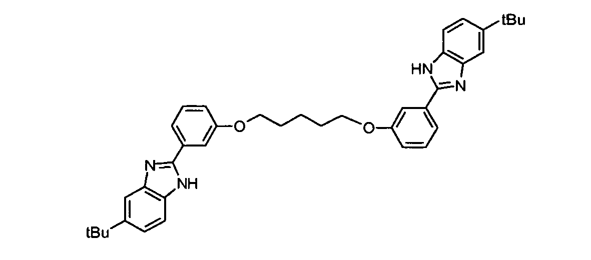 |
| **19** | 2,2'-[1,3-propanediylbis(oxy-1,2-phenylene)]bis-5-bromo-1H-benzimidazole | 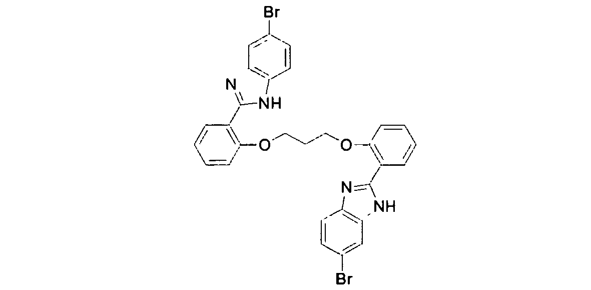 |
| **20** | 2,2'-[1,4-butanediylbis(oxy-1,3-phenylene)]bis-5-bromo-1H-benzimidazole | 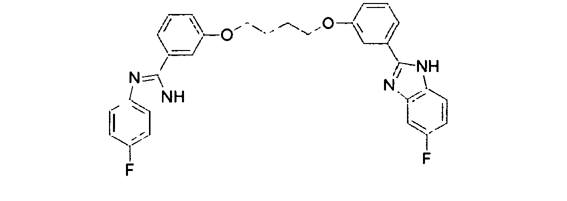 |
| **21** | 2,2'-[1,4-butanediylbis(oxy-1,3-phenylene)]bis-5-diethylamino-1 H-benzimidazole | 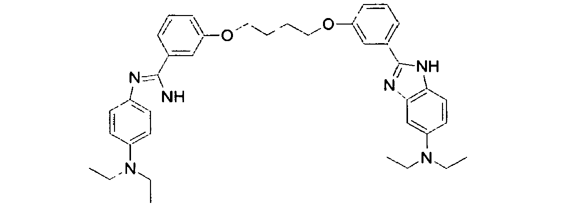 |
| **22** | 2,2'-[1,2-ethanediylbis(oxy-1,4-phenylene)]bis-5-propoxy-1H-benzimidazole | 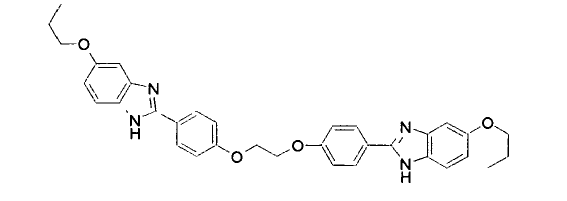 |
| **23** | Diethyl 2,2'-[1,2-ethanediylbis(oxy-1,4-phenylene)]bis-1 H-benzimidazole-5-carboxylate | 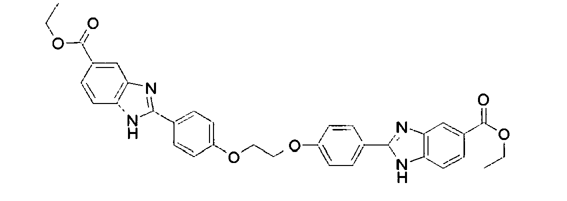 |
| **24** | 2,2'-[1,7-heptanediylbis(oxy-1,3-phenylene)]bis-5-dodecoxy-1H-benzimidazole | 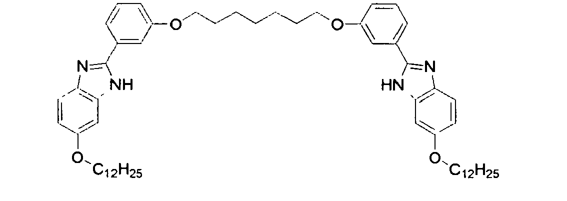 |
| **25** | Dioctyl 2,2'-[1,5-pentanediylbis(oxy-1,2-phenylene)]bis-1 H-benzimidazole-5-carboxylate | 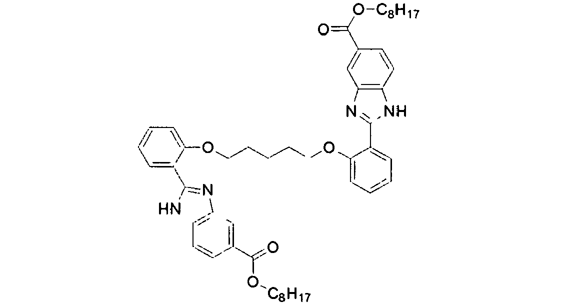 |

### Malaria: In vitro drug screening model against Plasmodium falciparum

### Parasite and cell cultures

Two strains of *P. falciparum* were used: 1/ GHA strain (*P.falGHA*), derived from a Ghanese patient and chloroquine sensitive and 2/ W-2 strain (*P.falW2*) from CDC/Indochina III and resistant to chloroquine, quinine, pyrimethamine, but susceptible to mefloquine. The strains were maintained in RPMI-1640 medium supplemented with 0.37 mM hypoxanthine, 25 mM Hepes, 25 mM NaHCO₃, and 10% O+ human serum together with 2-4% washed human O+ erythrocytes (Adaptation of the procedures described by Trager, W.; Jensen J. B. Science 1976, 193, 673-677). All cultures and assays were conducted at 37°C under an atmosphere of 4% CO₂, 3% O₂ and 93% N₂.

### Compound solutions/dilutions

Compound stock solutions were prepared in 100% DMSO at 20 mM or mg/ml (Concentrations are standard expressed in molar concentrations). The compounds were serially pre-diluted (2-fold or 4- fold) in DMSO followed by a further (intermediate) dilution in demineralized water to assure a final in-test DMSO concentration of <1%.

### Drug sensitivity assays

Assays were performed in sterile 96-well microtiter plates, each well containing 10 µl of the watery compound dilutions together with 190 µl of the malaria parasite inoculum (1% parasitaemia, 2% HCT). Parasite growth was compared to untreated-infected (100% growth) and uninfected-controls (0% growth). After 72h incubation at 37°C, plates were frozen at -20°C. After thawing, 20 µl of each well was transferred into another plate together with 100 µl Malstat reagent (Lactate dehydrogenase (LDH)-test, according to Makler et al.: Am.J.Trop Med. Hyg, 48, 7339-741, 1993) and 20 µl of a 1/1 mixture of PES (phenazine ethosulfat, 0.1 mg/ml) and NBT (NitroBlue Tetrazolium Grade III, 2 mg/ml). The plates were kept out of light for 2 hours and change in color was measured spectrophotometrically at 655 nm. The results were expressed as % reduction in parasitaemia compared to control wells. For each compound, an IC₅₀ was calculated.

### Primary screen

The GHA strain was used. Compounds were tested at 5 concentrations (64 - 16 - 4 - 1 and 0.25 µM or µg/ml). Artesunate (IC₅₀ = 0.005 + 0.004 µM) and chloroquine (IC₅₀ = 0.05 + 0.08 µM) were included as reference drugs. Some of the compounds were evaluated in a secondary screening.

### Secondary screen

Additional strains (GHA, W2, D6, NF54) were included and the IC₅₀-values were determined using an extended dose range (2-fold compound dilutions). Artesunate and chloroquine were included as reference drugs.

The results are shown in Table 2. Compounds for use in the invention were not cytotoxic.

**Table 2**

| | **IC₅₀ (µg / ml) (score)** |
|---|---|
| Chloroquine | 0.04 |
| Compound 1 | 16.58 (1) |
| Compound 3 | 0.49 (3) |
| Compound 6 | 3.91 (2) |
| Compound 7 | 8.31 (2) |

The compounds tested were active against *P*. *falciparum.* Therefore the compounds represent an interesting class of anti-malarial agents.

Compared to chloroquine some of the compounds, such as compound 3, were particularly selective since not active against some other strains of parasites.

## Claims

1. Use of at least one Compound of formula I or a pharmaceutically acceptable salt or solvate thereof for the preparation of a medicament for the prevention and/or treatment of malaria, 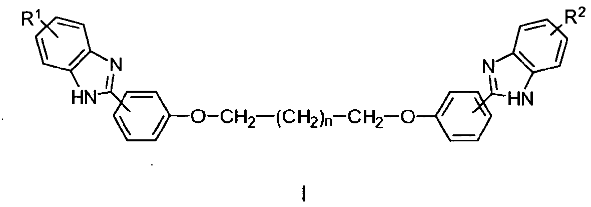 wherein n is an integer selected from 0 to 10, and
R¹ and R² are each independently selected from hydrogen, halogen, hydroxyl, alkyl, alkoxy, amino, alkylamino, alkylcarbonylamino, alkylcarbonyloxy, formyl, alkylcarbonyl, alkyloxycarbonyl, halocarbonyl, haloalkyl, haloalkoxy, carbamoyl, cyano, nitro, sulfo, or a carboxyl group.

2. Use according to claim 1, wherein n is 0, 1, 2, 3, 4, 5, 6 or 7.

3. Use according to claim 1 or 2, wherein R¹ and R² are each independently hydrogen, hydroxyl, halogen, alkyl, alkylamino, alkoxy, haloalkyl, haloalkoxy, alkyloxycarbonyl, alkylcarbonyl, amino, cyano, nitro or a carboxyl group.

4. Use according to any of Claims 1 to 3, wherein the at least one Compound of formula I is used in combination with at least one additional drug selected from the group consisting of chloroquine, artemesin, qinghaosu, 8-aminoquinoline, amodiaquine, arteether, artemether, artemisinin, artesunate, artesunic acid, artelinic acid, atovoquone, azithromycine, biguanide, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, primaquine, proguanil, pyrimethamine, pyronaridine, quinine, quinidine, quinacrineartemisinin, sulfonamides, sulfones, sulfadoxine, sulfalene, tafenoquine, tetracycline, tetrandine, triazine, salts or mixture thereof.

5. Use according to any of Claims 1 to 4, for the treatment of drug-resistant malaria.

6. Use according to any of Claims 1 to 5, wherein said malaria is selected from *Plasmodium falciparum* malaria, *P*. *vivax* malaria, *P. ovale* malaria, or *P. malariae* malaria.

7. A compound of formula I or a pharmaceutically acceptable salt or
solvate thereof 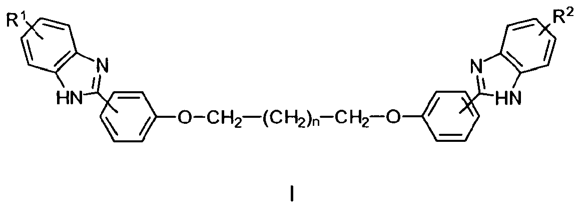 wherein
n is an integer selected from 0 to 10, and
R¹ and R² are each independently selected from hydrogen, halogen, hydroxyl, alkyl, alkoxy, amino, alkylamino, alkylcarbonylamino, alkylcarbonyloxy, formyl, alkylcarbonyl, alkyloxycarbonyl, halocarbonyl, haloalkyl, haloalkoxy, carbamoyl, cyano, nitro, sulfo,
or a carboxyl group,
for use in the therapeutic and/or prophylactic treatment of malaria.

8. The compound for use according to claim 7, wherein n is 0, 1, 2, 3, 4, 5, 6 or 7.

9. The compound for use according to claim 7 or 8, wherein R¹ and R² are each independently hydrogen, hydroxyl, halogen, alkyl, alkoxy, haloalkyl, haloalkoxy, alkylcarbonyl, amino, alkylamino, alkyloxycarbonyl, cyano, nitro, or a carboxyl group.

10. The compound for use according to any of Claims 7 to 9, to be administered in combination with at least one additional drug selected from the group consisting of chloroquine, artemesin, qinghaosu, 8-aminoquinoline, amodiaquine, arteether, artemether, artemisinin, artesunate, artesunic acid, artelinic acid, atovoquone, azithromycine, biguanide, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, Primaquine, proguanil, pyrimethamine, pyronaridine, quinine, quinidine, quinacrineartemisinin, Sulfonamides, sulfones, sulfadoxine, sulfalene, tafenoquine, tetracycline, tetrandine, triazine, salts or mixture thereof.

11. The compound for use according to any of Claims 7 to 10, for use in the treatment of an infection with *Plasmodium falciparum, P. vivax, P. ovale,* or *P. malariae.*

12. The compound for use according to any of Claims 7 to 11, to be administered after the subject has been exposed to the malaria parasite.

13. The compound for use according to any of Claims 7 to 12, wherein the malaria parasite is a drug-resistant malarial strain.

14. The compound for use according to any of Claims 7 to 13, to be administered before the subject travels to a country where malaria is endemic.

## Patentansprüche

1. Verwendung von zumindest einer Verbindung von Formel I oder eines pharmazeutisch annehmbares Salzes oder Solvats davon für die Herstellung eines Medikaments zur Prävention und/oder Behandlung von Malaria, 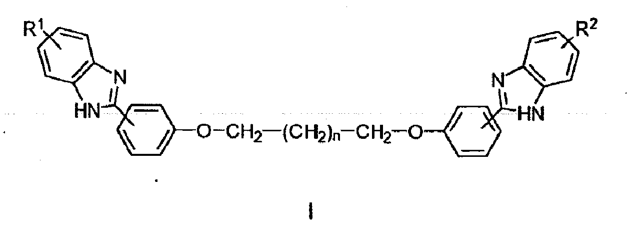 wobei n eine aus 0 bis 10 ausgewählte ganze Zahl ist, und
R¹ und R² jeweils unabhängig aus Wasserstoff-, Halogen-, Hydroxy-, Alkyl-, Alkoxy-, Amino-, Alkylamino-, Alkylcarbonylamino-, Alkylcarbonyloxy-, Formyl-, Alkylcarbonyl-, Alkyloxycarbonyl-, Halogencarbonyl-, Halogenalkyl-, Halogenalkoxy-, Carbamoyl-, Cyano-, Nitro-, Sulfo- oder einer Carboxylgruppe ausgewählt sind.

2. Verwendung nach Anspruch 1, wobei n 0, 1, 2, 3, 4, 5, 6 oder 7 ist.

3. Verwendung nach Anspruch 1 oder 2, wobei R¹ und R² jeweils unabhängig Wasserstoff-, Hydroxy-, Halogen-, Alkyl-, Alkylamino-, Alkoxy-, Halogenalkyl-, Halogenalkoxy-, Alkyloxycarbonyl-, Alkylcarbonyl-, Amino-, Cyano-, Nitro- oder eine Carboxylgruppe sind.

4. Verwendung nach einem der Ansprüche 1 bis 3, wobei die zumindest eine Verbindung von Formel I in Kombination verwendet wird mit zumindest einem weiteren Wirkstoff, der aus der aus Chloroquin, Artemesin, Quinghaosu, 8-Aminochinolin, Amodiaquin, Arteether, Artemether, Artemisinin, Artesunat, Artesunsäure, Artelinsäure, Atovoquone, Azithromycin, Biguanid, Chloroquin-Phosphat, Chlorproguanil, Cycloguanil, Dapson, Desbutyl-Halofantrin, Desipramin, Doxycyclin, Dihydrofolat-Reduktase-Inhibitoren, Dipyridamol, Halofantrin, Haloperidol, Hydroxychloroquin-Sulfat, Imipramin, Mefloquin, Penfluridol, Phospholipid-Hemmer, Primaquin, Proguanil, Pyrimethamin, Pyronaridin, Chinin, Chinidin, Quinacrin-Artemisinin, Sulfonamide, Sulfone, Sulfadoxin, Sulfalene, Tafenoquin, Tetracyclin, Tetrandin, Triazin, Salze oder Mischung davon bestehenden Gruppe ausgewählt ist.

5. Verwendung nach einem der Ansprüche 1 bis 4, für die Behandlung von wirkstoffresistenter Malaria.

6. Verwendung nach einem der Ansprüche 1 bis 5, wobei die Malaria aus *Plasmodium falciparum* Malaria, *P. vivax* Malaria, *P*. *ovale* Malaria oder *P. malariae* Malaria ausgewählt ist.

7. Eine Verbindung von Formel I oder ein pharmazeutisch annehmbares Salz oder Solvat
davon 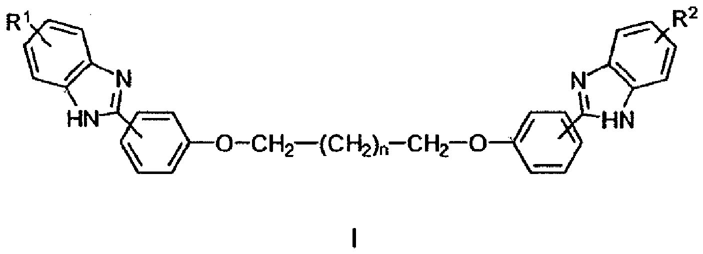 wobei
n eine ganze Zahl von 0 bis 10 ist und
R¹ und R² jeweils unabhängig aus Wasserstoff-, Halogen-, Hydroxy-, Alkyl-, Alkoxy-, Amino-, Alkylamino-, Alkylcarbonylamino-, Alkylcarbonyloxy-, Formyl-, Alkylcarbonyl-, Alkyloxycarbonyl-, Halogencarbonyl-, Halogenalkyl-, Halogenalkoxy-, Carbamoyl-, Cyano-, Nitro-, Sulfo- oder einer Carboxylgruppe ausgewählt sind,
zur Verwendung für die therapeutische und/oder prophylaktische Behandlung von Malaria.

8. Die Verbindung zur Verwendung nach Anspruch 7, wobei n 0, 1, 2, 3, 4, 5, 6 oder 7 ist.

9. Die Verbindung zur Verwendung nach Anspruch 7 oder 8, wobei R¹ und R² jeweils unabhängig Wasserstoff, Hydroxy, Halogen, Alkyl, Alkoxy, Halogenalkyl, Halogenalkoxy, Alkylcarbonyl, Amino-, Alkylamino, Alkyloxycarbonyl, Cyano, Nitro, oder eine Carboxylgruppe sind.

10. Die Verbindung zur Verwendung nach einem der Ansprüche 7 bis 9, zu verabreichen in Kombination mit zumindest einem weiteren Wirkstoff, der aus der aus Chloroquin, Artemesin, Quinghaosu, 8-Aminochinolin, Amodiaquin, Arteether, Artemether, Artemisinin, Artesunat, Artesunsäure, Artelinsäure, Atovoquone, Azithromycin, Biguanid, Chloroquin-Phosphat, Chlorproguanil, Cycloguanil, Dapson, Desbutyl-Halofantrin, Desipramin, Doxycyclin, Dihydrofolat-Reduktase-Inhibitoren, Dipyridamol, Halofantrin, Haloperidol, Hydroxychloroquin-Sulfat, Imipramin, Mefloquin, Penfluridol, Phospholipid-Inhibitoren, Primaquin, Proguanil, Pyrimethamin, Pyronaridin, Chinin, Chinidin, Quinacrin-Artemisinin, Sulfonamide, Sulfone, Sulfadoxin, Sulfalene, Tafenoquin, Tetracyclin, Tetrandin, Triazin, Salze oder Mischungen davon bestehenden Gruppe ausgewählt ist.

11. Die Verbindung zur Verwendung nach einem der Ansprüche 7 bis 10, zur Verwendung in der Behandlung einer Infektion mit *Plasmodium falciparum, P. vivax, P. ovale* oder *P*. *malariae.*

12. Die Verbindung zur Verwendung nach einem der Ansprüche 7 bis 11, zu verabreichen, nachdem das Subjekt dem Malariaparasit ausgesetzt worden ist.

13. Die Verbindung zur Verwendung nach einem der Ansprüche 7 bis 12, wobei der Malariaparasit ein wirkstoffresistenter Malariastamm ist.

14. Die Verbindung zur Verwendung nach einem der Ansprüche 7 bis 13, zu verabreichen, bevor das Subjekt in ein Land reist, in dem Malaria endemisch ist.

## Revendications

1. Utilisation d'au moins un composé de la formule I ou d'un sel ou solvate pharmaceutiquement acceptable de ce dernier pour la préparation d'un médicament pour la prévention et/ou le traitement du paludisme, 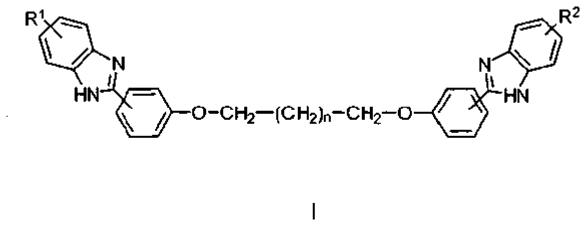 dans laquelle n est un nombre entier sélectionné dans la plage de 0 à 10, et
R¹ et R² sont chacun sélectionnés indépendamment parmi les éléments hydrogène, halogène, hydroxyle, alkyle, alkoxy, amino, alkylamino, alkylcarbonylamino, alkylcarbonyloxy, formyle, alkylcarbonyle, alkyloxycarbonyle, halocarbonyle, haloalkyle, haloalkoxy, carbamoyle, cyano, nitro, sulfo, ou un groupe carboxylique.

2. Utilisation selon la revendication 1, dans laquelle n est 0, 1, 2, 3, 4, 5, 6 ou 7.

3. Utilisation selon la revendication 1 ou 2, dans laquelle R¹ et R² sont chacun sélectionnés indépendamment parmi les éléments hydrogène, hydroxyle, halogène, alkyle, alkylamino, alkoxy, haloalkyle, haloalkoxy, alkyloxycarbonyle, alkylcarbonyle, amino, cyano, nitro ou un groupe carboxylique.

4. Utilisation selon l'une quelconque des revendications 1 à 3, dans laquelle le au moins un composé de formule I est utilisé en combinaison avec au moins un médicament additionnel sélectionné dans le groupe constitué de chloroquine, artémésine, qinghaosu, 8-aminoquinoline, amodiaquine, artééther, artéméther, artémisinine, artésunate, acide artésunique, acide artélinique, atovoquone, azithromycine, biguanide, phosphate de chloroquine, chlorproguanile, cycloguanile, dapsone, desbutyl halofantrine, désipramine, doxycycline, inhibiteurs de dihydrofolate réductase, dipyridamole, halofantrine, halopéridol, sulfate d'hydroxychloroquine, imipramine, méfloquine, penfluridol, inhibiteurs de phospholipide, primaquine, proguanile, pyriméthamine, pyronaridine, quinine, quinidine, quinacrineartémisinine, sulfonamides, sulfones, sulfadoxine, sulfalène, tafénoquine, tétracycline, tétrandine, triazine, sels ou un mélange de ces derniers.

5. Utilisation selon l'une quelconque des revendications 1 à 4, pour le traitement de paludisme résistant aux médicaments.

6. Utilisation selon l'une quelconque des revendications 1 à 5, dans laquelle ledit paludisme est sélectionné parmi le paludisme *Plasmodium falciparum,* le paludisme *P. vivax,* le paludisme *P. ovale,* ou le paludisme *P. malariae.*

7. Composé de la formule I ou sel ou solvate pharmaceutiquement acceptable de ce dernier, 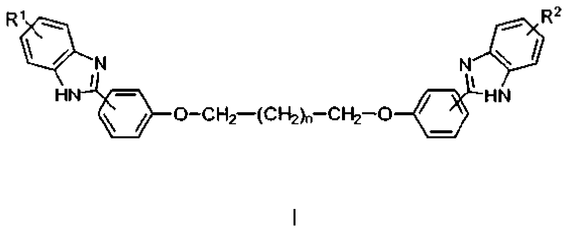 dans lequel n est un nombre entier sélectionné dans la plage de 0 à 10, et
R¹ et R² sont chacun sélectionnés indépendamment parmi les éléments hydrogène, halogène, hydroxyle, alkyle, alkoxy, amino, alkylamino, alkylcarbonylamino, alkylcarbonyloxy, formyle, alkylcarbonyle, alkyloxycarbonyle, halocarbonyle, haloalkyle, haloalkoxy, carbamoyle, cyano, nitro, sulfo, ou un groupe carboxylique, pour utilisation dans le traitement thérapeutique et/ou prophylactique du paludisme.

8. Composé selon la revendication 7, dans lequel n est 0, 1, 2, 3, 4, 5, 6 ou 7.

9. Composé pour utilisation selon la revendication 7 ou 8, dans lequel R¹ et R² sont chacun sélectionnés indépendamment parmi les éléments hydrogène, hydroxyle, halogène, alkyle, alkoxy, haloalkyle, haloalkoxy, alkylcarbonyle, amino, alkylamino, alkyloxycarbonyle, cyano, nitro ou un groupe carboxylique.

10. Composé pour utilisation selon l'une quelconque des revendications 7 à 9, destiné à être administré en combinaison avec au moins un médicament additionnel sélectionné dans le groupe constitué de chloroquine, artémésine, qinghaosu, 8-aminoquinoline, amodiaquine, artééther, artéméther, artémisinine, artésunate, acide artésunique, acide artélinique, atovoquone, azithromycine, biguanide, phosphate de chloroquine, chlorproguanile, cycloguanile, dapsone, desbutyl halofantrine, désipramine, doxycycline, inhibiteurs de dihydrofolate réductase, dipyridamole, halofantrine, halopéridol, sulfate d'hydroxychloroquine, imipramine, méfloquine, penfluridol, inhibiteurs de phospholipide, primaquine, proguanile, pyriméthamine, pyronaridine, quinine, quinidine, quinacrineartémisinine, sulfonamides, sulfones, sulfadoxine, sulfalène, tafénoquine, tétracycline, tétrandine, triazine, sels ou un mélange de ces derniers.

11. Composé pour utilisation selon l'une quelconque des revendications 7 à 10, pour utilisation dans le traitement d'une infection par *Plasmodium falciparum, P. vivax, P. ovale,* ou *P. malariae.*

12. Composé pour utilisation selon l'une quelconque des revendications 7 à 11, destiné à être administré après que le sujet a été exposé au parasite du paludisme.

13. Composé pour utilisation selon l'une quelconque des revendications 7 à 12, dans lequel le parasite du paludisme est une variante de paludisme résistante aux médicaments.

14. Composé pour utilisation selon l'une quelconque des revendications 7 à 13, destiné à être administré avant que le sujet ne voyage dans un pays ou le paludisme est endémique.
